# EUROPEAN PATENT APPLICATION

(11) **EP 2 133 695 A1**
(43) Date of publication of application: **16.12.2009**
(21) Application number: 08158163.9
(22) Date of filing: 12.06.2008
(51) Int. Cl.: G01N 33/543, G01N 33/78

(54) **Parathyroid hormone assay**

(71) Applicant: Koninklijke Philips Electronics N.V., 5621 BA Eindhoven (NL)
(72) Inventor: The designation of the inventor has not yet been filed
(74) Representative: Damen, Daniel Martijn

(57) **Abstract**

The present invention relates to one-step sandwich immunoassays for the detection of parathyroid hormone (PTH) based on the manipulation of antibodies labelled with magnetic particles under improved assay conditions.

## Description

### FIELD OF THE INVENTION

The invention relates to methods and tools for determining Parathyroid Hormone in a sample, which methods and tools allow for an increased sensitivity in a shorter time-frame.

### BACKGROUND OF THE INVENTION

Parathyroid disorders occur when the parathyroid glands over- or underproduce Parathyroid Hormone (PTH), which is a hormone that regulates calcium absorption and phosphorus levels in the body. A common disorder is hyperparathyroidism (1 in 1000 of the general population, and an even higher frequency in postmenstrual women) in which PTH levels are elevated, resulting in a high concentration of calcium levels in blood because of the removal of calcium from bones, the enhanced absorption of calcium from the intestines, and the decreased release of calcium into the urine. This can result in constipation, nausea, vomiting, fatigue, and other symptoms. It can also lead to kidney stones and weakening of the bones (osteoporosis).

Blood tests for PTH are used to diagnose parathyroid disorders or to identify the underlying cause of abnormal calcium levels in the body. In addition, PTH measurements are also performed in surgery upon removal of enlarged parathyroid glands or parathyroid adenomas to determine whether the surgery has removed all target tissue or whether additional tissue needs to be removed. Two-site radioimmunoassays and ELISA are the common techniques used to measure the level of PTH in blood.

The technical demands for PTH tests are high as it is desirable to detect in patient blood samples PTH concentrations in the picomolar range. It is also desirable to perform these tests rapidly with an easy to use system. This is especially important for intraoperative PTH determinations as the surgeon relies on real-time assay results to make surgical decisions.

State of the art immunoassays use magnetic particles as detection labels, which enables magnetic actuation and reduces the assay time. In these assays, magnetic particles are sterically hindered due to their large dimensions and do not bind to a surface with molecular receptors as easily as molecular labels (such as radioactive iodine, enzymatic label). Moreover, because particles are magnetised during the assay, the duration of contact between particles increases which enhances the probability that the particle labels irreversibly aggregate and quantitative information is lost. This can be a significant problem when larger magnetic particles (>200nm) are used as they are more prone to form irreversible clusters. The aggregation can be further enhanced by the physicochemical properties of the ligand, as is the case for PTH.

The use of large magnetic particles also has advantages. In some cases it is desirable to use larger labels as this improves the speed at which particles can be actuated through a fluid and thus the speed at which the assay can be performed. Additionally, with certain detection technologies the larger the particle size is, the larger the detected signal per label.

### SUMMARY OF THE INVENTION

Particular and preferred aspects of the invention are set out in the accompanying independent and dependent claims. Features from the dependent claims may be combined with features of the independent claims and with features of other dependent claims as appropriate and not merely as explicitly set out in the claims.

According to one aspect of the invention, methods are provided for detecting parathyroid hormone (PTH) in a sample comprising the steps of,
a) contacting the sample with a soluble, first anti-PTH antibody coupled to a magnetic particle with a diameter between 100 and 1000 nm,
b) manipulating the magnetic particles with a magnetic field so as to bring the particles into contact with a detection surface comprising a second anti-PTH antibody immobilised on the detection surface,
c) detecting the binding of the magnetic particles on the detection surface wherein, steps a) and b) are performed in an alkaline buffer between pH 8.2 and 10.6 (e.g. between pH 8.6-10.2, pH 8.8-10.0, pH 9.0-9.8 or pH 9.2-9.6).

In particular embodiments the size range of magnetic particles has a lower limit of 150 or 200 nm. In other particular embodiments the size range of magnetic particles has an upper limit of 500 or 750 nm.

In particular embodiments, the alkaline buffer has a lower limit of pH 8.4, 8.6, 8.8, 9.0, 9.2 or 9.3. In other particular embodiments, the alkaline buffer has an upper limit of pH 9.5, 9.6, 9.8, 10.0, 10.2 or 10.4.

According to particular embodiments the manipulation comprises the repeated application of a magnetic field manipulating magnetic particles towards the sensor surface.

According to other particular embodiments, the manipulation comprises the application of an additional magnetic field generating a translational and/or rotational movement of magnetic particles over the sensor surface.

According to a particular embodiment, the buffer is a borate buffer.

According to other particular embodiments, the buffer further comprises BSA or IgG and/or a detergent.

According to yet other embodiments the detection of the magnetic particles is performed based on the magnetic properties of these particles or alternatively the detection of the magnetic particles is performed based on the optical properties of the particles.

In yet other particular embodiments the magnetic particles further comprise an optical label and the detection is performed based on the detection of the optical label.

Another aspect of the invention relates to the use of a buffer with a pH of between 8.2 and 10.6 in a sandwich ELISA for PTH involving magnetic particles. Other suitable pH ranges have a lower limit of pH 8.4, 8.6, 8.8, 9.0, 9.2 or 9.3 or have an upper limit of 9.5, 9.6, 9.8, 10.0, 10.2 or 10.4. (e.g. between pH 8.6-10.2, pH 8.8-10.0, pH 9.0 - 9.8 or pH 9.2-9.6).

In particular embodiments the buffer is a borate buffer and further comprises BSA and/or IgG, optionally further comprises a detergent, such as Tween 60.

Another aspect of the invention relates to a composition comprising antibodies against PTH, wherein the antibodies are labelled with magnetic particles, and wherein the magnetic particles have a diameter between 100 and 1000 nm. Alternatively these particles have a diameter with a lower limit of 150 or 200 nm. Alternatively these particles have a diameter with an upper limit of 500 or 750 nm.

In particular embodiments the composition further comprises PEG or biotin.

In other particular embodiments the composition further comprises a buffer with a pH of between 8.2 and 10.6. Other suitable pH ranges have a lower limit of pH 8.4, 8.6, 8.8, 9.0, 9.2 or 9.3 or have an upper limit of 9.5, 9.6, 9.8, 10.0, 10.2 or 10.4. (e.g. pH 8.6-10.2, pH 8.8-10.0, pH 9.0 - 9.8 or pH 9.2-9.6).

The present invention describes a biosensor platform technology that is able to measure very sensitively the number of magnetic particle labels on a sensor surface using, e.g., a compact handheld device. When the labels come into the proximity of the surface via an immunoassay, the device can be used to sensitively detect the protein concentration in a sample. The device when applied to in vitro diagnostics, allows testing to be done directly at the point of care.

The present invention describes a one-step magnetic particle label based immunoassay for PTH that enables the rapid quantitative measurement of PTH, which is performed in the above-described platform and which enables results to be obtained within minutes of sample-taking in demanding point of care environments such as in the operating room and physician's office.

Magnetic particles containing antibodies (especially polyclonal antibodies) at high concentrations are prone to irreversible aggregation. Embodiments of methods of the present invention whereby such irreversible aggregation is avoided include one or more of the following:
- the use of blocking methods in the preparation of magnetic particles covered with a monolayer or bilayer of antibody, to reduce irreversible aggregation and maintain high binding activity;
- the use of buffer compositions and buffer additives to reduce the irreversible aggregation of magnetic particles, especially under the repeated influence of a magnetic field; the use of specific magnetic actuation methods to enhance the efficiency of particles binding to a sensor surface, and to reduce irreversible cluster formation; and the use of adapted methods for the immobilisation of antibodies on inorganic surfaces.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### BRIEF DESCRIPTION OF THE FIGURES.

Fig. 1 shows a schematic overview of a one step PTH assay in accordance with an embodiment of the invention. 1: PTH; 2: first anti-PTH antibody; 3: magnetic particle; 4: second anti-PTH antibody; 5: sensor surface.
Fig. 2 shows in accordance with an embodiment of the invention, a dose response curve of PTH detection using 300 nm (squares) and 500 nm (triangles) magnetic particles.

In the different figures, the same reference signs refer to the same or analogous elements.

The present invention will be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The following terms or definitions are provided solely to aid in the understanding of the invention. These definitions should not be construed to have a scope less than understood by a person of ordinary skill in the art.

### DETAILED DESCRIPTION OF THE INVENTION

### Definitions

The term **"first antibody"** as used herein refers to an antibody capable of specifically binding to an analyte which is coupled to a magnetic particle and which is in solution. Such antibodies are also known in the art as "tracer antibodies".

The term **"second antibody"** as used herein refers to an antibody capable of specifically binding to the same analyte as the first antibody, which is immobilised on a detection surface. Such antibodies are also known in the art as "capture antibodies".

The first and second antibody are selected such that the analyte of interest, i.e. in the present case PTH, can bind both the first and the second antibody simultaneously.

The term "detection surface" as used herein refers to a surface to which antibodies can be coupled and which allows the detection of a label in its vicinity. Typically, the detection surface is a solid, uniform surface. The detection can be a sensor surface, i.e. a surface which is involved in detection. Alternatively, the sensor can be located in the vicinity e.g. under the detection surface, allowing detection of labels present close to the detection surface.

The present invention discloses methods wherein mammalian, more particular human Parathyroid Hormone (PTH) is detected using antibodies.

In particular embodiments of the invention two antibodies directed against PTH are used. The anti-PTH antibodies which are used in methods of the present invention can be polyclonal antibodies raised in a host of choice, or can be monoclonal antibodies.

In particular embodiments one or both of the anti-PTH antibodies are goat polyclonal antibodies, which have been raised against PTH and optionally have been affinity purified using PTH. In more particular embodiments each of the two antibodies has been raised against a different PTH epitope and/or has been affinity purified using a different PTH peptide. In further particular embodiments, one the anti-PTH antibodies has been raised and/or affinity purified using the peptide containing amino acid 1-34 and the other antibody has been raised and/or affinity purified using the peptide containing amino acids 39-84 of human PTH. In more particular embodiments, antibodies against amino acid 39-84 of PTH are used as the second antibodies, i.e. the antibodies immobilised on a sensor surface and antibodies against amino acids 1-34 of PTH are used as first antibodies, i.e. bound to magnetic particles. However, other combinations of first and second antibodies are similarly applicable. Moreover the use of functional fragments of antibodies or recombinant antibody fragments is also envisaged.

The assays of the present invention are sandwich assays wherein a first antibody is in solution and bound to a label, and a second unlabelled antibody is immobilised on a sensor surface. In the methods and compositions of the present invention, the first antibody is bound to a magnetic particle. This magnetic particle is used for manipulation with a magnetic field.

The nature of the magnetic particle used in the context of the present invention is not critical. Suitable magnetic particles include completely inorganic particles and particles which are a mixture of an inorganic and an organic material (e.g. a polymer).

Magnetic particles are commercially available from e.g. Dynal, Estapor, Seradyn and are widely used in biological analysis that are available from several diagnostic companies (Roche, Bayer, Johnson & Johnson, Abbott, BioMerieux, etc.).

Attachment of antibodies or other compounds to the surface of magnetic particles can be performed by methods described in the art. For instance, the particles may carry functional groups such as hydroxyl, carboxyl, aldehyde or amino groups. These may in general be provided, for example, by treating uncoated monodisperse, superparamagnetic particles, to provide a surface coating of a polymer carrying one of such functional groups, e. g. polyurethane together with a polyglycol to provide hydroxyl groups, or a cellulose derivative to provide hydroxyl groups, a polymer or copolymer of acrylic acid or methacrylic acid to provide carboxyl groups or an aminoalkylated polymer to provide amino groups. US Patent 4,654,267 describes the introduction of many such surface coatings. Other coated particles may be prepared by modification of the particles according to the US4,336,173, US4,459,378 and US4,654,267. For example, macroreticular porous polymer particles, prepared from styrene-divinylbenzene and with a diameter of 3.15 µm were treated with HNO₃ to introduce-NO₂ groups at the surface of the pores. Then the particles were dispersed in an aqueous solution of Fe. The Fe²⁺ is oxidized by the NO₂ groups which leads to precipitation of insoluble iron oxy-hydroxy compounds inside the pores. After heating the iron exists as finely divided grains of magnetic iron oxides throughout the volume of the porous particles The NO₂ groups are reduced by the reaction with Fe to NH₂ groups. To fill up the pores and to introduce the desired functional groups at the surface s, different monomers are caused to polymerize in the pores and at the surface. In the case of a preferred type of particle, the surface carries-OH groups connected to the polymeric backbone through (CH₂CH₂O)₈₋₁₀ linkages. Other preferred particles carry -COOH groups obtained through polymerization of methacrylic acid. For example, the NH₂ groups initially present in the particles may be reacted with a di-epoxide as described in US4,654,267 followed by reaction with methacrylic acid to provide a terminal vinyl grouping. Solution copolymerization with methacrylic acid yields a polymeric coating carrying terminal carboxyl groups. Similarly, amino groups can be introduced by reacting a diamine with the above product of the reaction with a diepoxide, while reaction with a hydroxylamine such as aminoglycerol introduces hydroxy groups. The coupling of a bio active molecule to a particle can be irreversible but can also be reversible by the use of a linker molecule for the crosslinking between particle and bioactive molecule. Examples of such linkers include peptides with a certain proteolytic recognition site, oligonucleotide sequences with a recognition site for a certain restriction enzyme, binding partners such as streptavdin/biotin, or chemical reversible crosslinking groups as those comprising a reducible disulfide group. A variety of reversible crosslinking groups can be obtained from Pierce Biotechnology Inc. (Rockford, IL, USA).

Magnetic particles are commercially available in various sizes, ranging from nanometers to micrometers. Magnetic particles of between 100 nm and 1000 nm are envisaged to be suitable for use in the context of the invention. However, in particular embodiments the diameter of the magnetic particle is 200 nm or more. In other particular embodiments the diameter of the magnetic particle 500 nm or less. Similarly, the shape of the particles (spheres, spheroids, rods) is not critical.

The methods of the present invention envisage the movement and (direct or indirect) detection of magnetic particles. In particular embodiments, the magnetic particles are detected in a detection region based on their magnetic properties. In this regard, the use of particles with different types of magnetic properties (magnetic, paramagnetic, superparamagnetic, ferromagnetic, i.e. any form of magnetism which has a magnetic dipole in a magnetic field, either permanently or temporarily) is envisaged. Additionally or alternatively, the e.g. optical or electric properties of the magnetic particle are used for detection.

Additionally or alternatively it is envisaged that magnetic particles can be detected based on the presence of a label either directly attached to the magnetic particle or indirectly bound to the particle through an analyte.

Accordingly, in particular embodiments, the magnetic particle used in methods and devices described herein is labelled with additional non-magnetic labels for detection of the magnetic particles. Labels can be attached to magnetic particles via the inorganic or via the organic component at the outside or can be incorporated into the particle.

Suitable labels in the context of the present invention are those labels which are classically used in *in vitro* assays such as, but not limited to, chromophoric groups, radioactive labels, electroluminescent, chemiluminescent, phosphorescent, fluorescent or reflecting labels.

In quantitative assays wherein magnetic particles are used, it is preferred that the magnetic particles remain as monodisperse as possible. This is a challenge especially when large (>100 or > 200nm) magnetic particles are employed and are subjected to a magnetic field. Typically, antibodies to PTH are coupled to magnetic particles (e.g. Ademtech, Dynal, Estapor etc) using e.g. reactive carboxyl groups on the particle surface or by streptavidin-biotin interactions. High antibody concentrations in such coupling solutions (>10 µg Ab /mg magnetic particle, most preferably about 50 µg Ab /mg) result in an increased sensitivity of the assay. However, magnetic particles containing such high antibody coverage have an enhanced tendency to cluster irreversibly in solution especially in the presence of PTH. In particular embodiments of methods according to the present invention, conditions are selected which reduce the aggregation of magnetic particles. These conditions include one or more of the following.

In particular embodiments the conditions selected such that the aggregation of magnetic particles during or after the coupling of antibodies to these particles is reduced include the adding one or more blocking agents to the magnetic particles after coupling. In particular embodiments, more particularly where the particles are streptavidin-coated (for the binding of the first antibody through biotin) the blocking agent is biotin and/or PEG. The addition of biotin (e.g. 1, 10 up to 100 µg/ml magnetic particle solution) or PEG 0.1 up to 3% after coupling of antibodies to magnetic particles has been observed to significantly reduce the amount of clusters formed and this both in the absence and in the presence of a magnetic field. This is demonstrated in Table 1 below. Whereas the blocking with PEG is particularly suitable for streptavidin/biotin coupled antibodies, it was observed that PEG equally has a positive effect and prevents clustering of magnetic particles functionalised with carboxyl groups.

**Table 1. Effect of blocking agents on the aggregation of streptavidin coated magnetic particles (200 nm diameter) covered with anti-PTH antibodies.**

| blocking agent | Buffering component | incubation on roller bank (min) | | | | | | | magnet |
|---|---|---|---|---|---|---|---|---|---|
| | | 0 | 15 | 30 | 45 | 60 | 75 | 90 | 3+ |
| - | PBS | - | - | + | + | 2+ | 3+ | +3 | 4+ |
| - | Borate | - | + | + | + | 3+ | 4+ | 4+ | 4+ |
| PEG | PBS | - | - | + | + | 2+ | 3+ | 4+ | 2+ |
| PEG | Borate | - | -# | + | + | + | + | + | |
| PVA | PBS | - | + | 4+ | 5+ | 5+ | | | |
| PVA | Borate | - | + | 4+ | 5+ | 5+ | | | |
| Biotin | PBS | - | -# | + | + | 2+ | 2+ | 2+ | 3+ |
| Biotin | Borate | - | -# | + | + | + | + | + | 2+ |

| | | | | | | | | | |
|---|---|---|---|---|---|---|---|---|---|
| - : no aggregates; +# probably not aggregated, single microparticles, but better visible that those of "-"; +: 1-4 particles/aggregate; 2+ : 1-15 particles/aggregate; 3+: 4-25 particles/aggregate + a few aggregates; 5+: huge aggregates. | | | | | | | | | |

In particular embodiments of the present invention, the conditions involve the use of improved buffer conditions during the assay to decrease the aggregation of magnetic particles.

It has been observed that the resuspension of magnetic particles in a high pH buffer maintains particles in monodisperse conditions, both in the presence or absence of a magnetic force. In particular embodiments, the high pH buffer is a buffer such as a borate buffer between pH 8.5 and pH 10.2 , typically between pH 8.5 and 9.8 (e.g. between 9.0 and 9.6, between pH 9.2 and 9.4 or between pH 9.3 and 9.5, e.g. pH 9.4 (pH 9.4). Other buffers which are envisaged to be suitable for obtaining the above-mentioned pH values between pH 8.2 and 10.6 are for example carbonate buffer, glycine buffer and CHES (2-(N-Cyclohexylamino)ethane Sulfonic Acid) buffer. The impact of buffer conditions on aggregation is shown in Table 2 below. The use of buffers with high pH prevents the aggregation of magnetic particles with anti-PTH first antibodies both in the absence or presence of a magnetic force (i.e. under assay conditions). This is particularly advantageous since the aggregation of magnetic particle with first antibodies increases when PTH is bound to these antibodies.

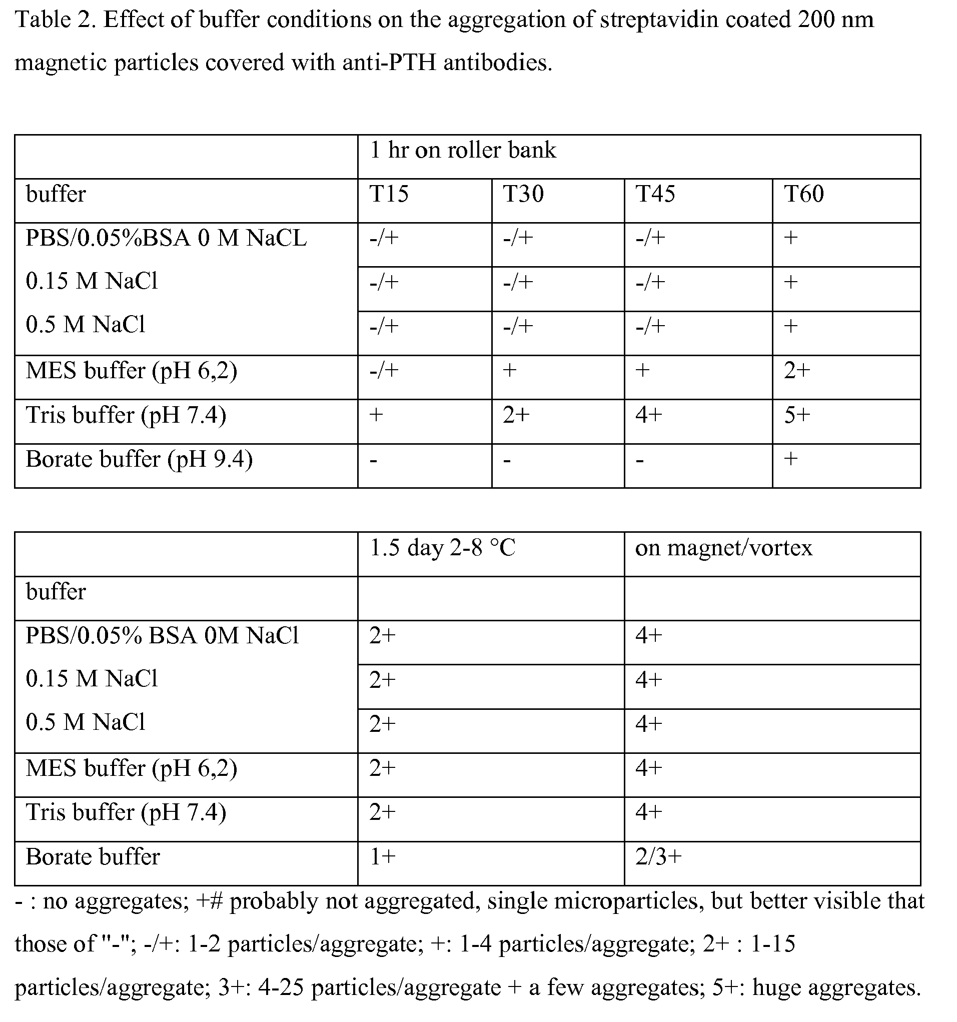

In particular embodiments of the present invention, the conditions involve the addition of proteins to the buffer. It has been observed that the monodisperse character of the magnetic particles is further improved by the addition of proteins such as IgG and BSA at a concentration of between about 0.01 and 3%, more particular between about 0.05-0.2 % IgG and/or about 0.1 to 1% BSA.

In particular embodiments of the present invention, the conditions involve the addition of ionic or non-ionic detergents to the assay buffer. Indeed, it has been observed that the monodisperse character of the magnetic particles is also improved by the addition of ionic or non-ionic detergents such as Chaps, SDS, Pluronic, and Tween 60 (Polyethylene glycol sorbitan monostearate). Table 3 shows that Tween 60 is most effective in reducing the formation of aggregates.

**Table 3. Effect of detergents on the aggregation of streptavidin coated 200 nm magnetic particles covered with anti-PTH antibodies.**

| | 1hr on roller bank | | | | on magnet / vortex |
|---|---|---|---|---|---|
| PBS/ 0.05% BSA + | T15 | T30 | T45 | T60 | |
| 1% Tween-20 | + | 4+ | 4+ | 4/5+ | 5+ |
| 1% Triton | + | 3+ | 4+ | 4+ | 3+ |
| 1% CHAPS | -/+ | -/+ | -/+ | - | 2+ |
| 1% Pluronic | - | - | - | - | + |
| 0.05 % SDS | 4+ | | - | 5+ | 4/5+ |
| 0.1 % SDS | -# | | -# | -# | + |
| 1% Zwittergent | + | | 3+ | 4+ | 3+ |
| 1% Tween-60 | - | | - | - | + |

| | | | | | |
|---|---|---|---|---|---|
| - : no aggregates; +# probably not aggregated, single microparticles, but better visible that those of "-"; -/+: 1-2 particles/aggregate; +: 1-4 particles/aggregate; 2+ : 1-15 particles/aggregate; 3+: 4-25 particles/aggregate + a few aggregates; 5+: huge aggregates | | | | | |

Assays in which one or more of the above-mentioned conditions for reducing aggregation are used show improved sensitivity and increased efficacy of detection. ELISA tests show that the above mentioned buffer components do not interfere with antigen-antibody binding. Although commercial buffers for the storage of magnetic particles also to some extent prevent aggregation of particles, the antigen-antibody binding itself is decreased in these storage buffers, when they are used during the assay itself.

In particular embodiments of the invention, at least two, more particularly at least three, most particularly all four of the conditions described above (blocking agent after coating of particles and all three buffer conditions) are applied. More particularly, at least two, particularly all three of the conditions relating to the assay buffer (high pH, presence of protein and ionic or non-ionic detergent) are used. In most particular embodiments, the PTH detection assays is carried out using a buffer comprising 100 mM Sodium borate pH 9.4, 150 mM NaCl, 0.5% BSA, 0.1% goat IgG, 1% Tween 60.

The conditions relating to the buffer described above are of particular interest for the buffer used during the PTH detection assay. Additionally, the high pH buffers of the present invention can also be added to the sample (e.g. human serum, plasma or blood, saliva, milk) to reduce clustering of magnetic particles with first antibodies present in these samples.

The efficiency and the nature of the coupling/immobilization of the first antibody on the magnetic label and/or the second antibody on the detection surface will also significantly affect the sensitivity of the PTH detection assay. Accordingly, in particular embodiments of the invention, conditions are applied wherein the coupling/immobilisation conditions of the anti-PTH antibodies are optimized. In particular embodiments, the immobilisation conditions of anti-PTH second antibodies on the inorganic surface (e.g. gold) of a sensor, are as described in example 2.

In particular embodiments the anti-PTH first antibodies are applied in an oriented way on a magnetic particle surface by using a randomly oriented first protein layer such as protein A, G, or Fc specific anti-IgG. Hereafter anti-PTH antibodies are immobilised on top via the Fc region of the anti-PTH antibody which now faces the particle, while the antigen binding region is exposed. Alternatively, the anti-PTH antibody is biotinylated at its Fc region and is then attached to streptavidin coated magnetic particles via the Fc region.

A further aspect of the invention relates to conditions for optimizing antigen-antibody interaction. Accordingly, in particular embodiments of the present invention, use is made of improved magnetic actuation methods so as to obtain a high sensitivity and increased efficacy. The probability that a magnetic particle with a first antibody can, through binding to PTH, bind to a second antibody on the surface of a sensor depends on the amount of PTH present in the sample. However, antigen-antibody binding requires not only the vicinity of the antigen to the antibody but also the appropriate orientation of the antigen and antibody with respect to each other. In order to increase the probability that PTH can encounter a second antibody on the sensor surface in the correct orientation so as to bind it, it is of interest to ensure that the magnetic particles are brought in the vicinity of the sensor surface and presented to the sensor surface in as many orientations as possible.

In particular embodiments of the methods described in the present invention, the optimization of antigen-antibody interaction is achieved by applying a magnetic field directed towards the detection surface and/or pulsed actuation forces to the magnetic particles carrying the first anti-PTH antibodies during the assay to ensure optimized contact with the detection surface.

Magnetic particles can be manipulated in different ways to optimise contact with the immobilized antibodies. In particular embodiments, magnetic actuation in the assay is performed as follows.

In a first step, the particles are rapidly attracted to the sensor surface in a "collection" step. This is ensured by applying a magnetic field in the direction of the sensor surface. In particular embodiments the magnetic field ensures that the magnetic particles have reached the sensor surface, for instance such as to reach at least 50%, 75% or 90% of monolayer formation on the surface, preferably 100% monolayer formation.

In a second step, the magnetic forces are removed and the particles are allowed to move over the surface with essentially unhindered translational as well as rotational degrees of freedom. After a certain time diffusion occurs and, in particular embodiments it is envisaged that the oriented magnetic field of the first step is once again applied. These steps can be repeated several times to ensure that all magnetic particles with PTH bound to first antibodies are bound to the second antibody on the detection surface. By this alternation of on/off of the magnetic field, pulsed actuation is obtained.

In alternative embodiments of the magnetic actuation conditions envisaged herein, the rotation and translation at the detection surface is not merely a result of passive diffusion in the absence of a magnetic field, but is actively ensured by the application of one or more magnetic fields which ensure the movement of magnetic particles over the detection surface.

In particular embodiments the magnetic force ensuring the movement of magnetic particles over the detection surface is ensured by pulsed actuation of the particles. This can involve e.g. alternating the direction of a magnetic field perpendicular to the detection surface or parallel to the detection surface or a combination of different fields with different orientations. Such methods are described e.g. in WO2007129275. The time and duration of each pulse is designed based on the particle size so as to optimally allow the particle to undergo at least one full rotation over its axis over the binding surface. In particular embodiments, the actuation forces are essentially perpendicular to the surface as strong forces parallel with the sensor surface can remove specifically bound magnetic particles.

In methods described herein it is optionally envisaged that, after the contacting of the magnetic particles with the detection surface through magnetic actuation, a magnetic force is applied directing the particles away from the detection surface to ensure the removal of unbound particles. In this way a washing step can be ensured.

It has been found that methods involving pulsed actuation alternated with translational and rotational movement of magnetic particles on the detection surface are significantly more efficient than methods which involve only a constant magnetic force attracting the particles to the detection surface. The pulsed actuation also reduces the probability that particles irreversibly aggregate as the amount of time that the particles are in contact with one another is also reduced.

Particular embodiments of the present invention relate to methods wherein one or more of the different conditions for optimising detection of PTH are combined. In further particular embodiments all of the above-described conditions improving the detection of PTH are combined.

As mentioned above the detection of magnetic particles at a detection surface can be ensured by any direct or indirect method known in the art. Particular detection methods are based on the magnetic properties of the particle such as GMR or on optical properties of the magnetic particles, such as detection with frustrated total internal reflection (FTIR). Miniaturised GMR sensor chips, integrated in disposable flow-cell cartridges, as described in e.g. Nellissen et al. (2007) in proceedings of the15th European Microelectronics and Packaging Conference p210-204, or in De Boer et al. (2007) Biosens. Bioelectron. 22, 9-10, are suitable for performing the methods of the present invention, and can detect a particle density of three 300 nm particles on a 1500 µm² chip surface.

The detection surface to which the second anti-PTH antibodies are bound in devices used in the methods of the invention is typically a specially derivatized surface to which molecules, more particularly antibodies or functional fragments thereof can be bound. Examples of suitable surfaces include, glass, metal, plastic, an organic crystal or an inorganic crystal (e. g. silicon), an amorphous organic or an amorphous inorganic material (e. g. silicon nitride, silicon oxide, silicon oxinitride, aluminum oxide). Suitable surface materials and linking chemistries are known to the person skilled in the art, and are described for instance in "Diagnostic Biosensor Polymers", by A. M. Usmani and N. Akmal, American Chemical Society, 1994 Symposium Book Series 556, Washington DC, USA, 1994, in "Protein Architecture, Interfacing Molecular Assemblies and Immobilization Biotechnology", edited by Y. Lvov and H.Mhwald (Marcel Dekker, New York, 2000), in "The Immunoassay Handbook" by David Wild (Nature Publishing Group, London, 2001, ISBN 1 -56159-270-6) or "Handbook of Biosensors and Electronic Noses. Medicine, Food and the Environment" by Kress-Rogers (ISBN 0-8493-8905-4). Supports for coupling proteins to coated and uncoated plastic and glass supports are disclosed in Angenendt et al. (2002; Anal Biochem. 309, 253-260).

Detection means suitable for use in the methods, systems and devices of the present invention are detection means capable of detecting the relevant signal such as, but not limited, to a magnetic signal, magnetoresistance, a Hall effect, an optical signal (reflection, absorption, scattering, fluorescence, chemiluminescence, RAMAN, FTIR, etc.). Such optical labels are known to the skilled person and include fluorescein dyes, such as 5- (and 6-) carboxy-4',5'-dichloro-2',7'-dimethoxy fluorescein, 5-carboxy-2',4',5',7'-tetrachlorofluorescein and 5-carboxyfluorescein, rhodamine dyes such as 5- (and 6-) carboxy rhodamine, 6-carboxytetramethyl rhodamine and 6-carboxyrhodamine X, phthalocyanines such as methyl, nitrosyl, sulphonyl and amino phthalocyanines, azo dyes, azomethines, cyanines and xanthines such as the methyl, nitro, sulphano and amino derivatives, and succinylfluoresceins. Other suitable labels are fluorophores from the group of cyanine dimers and monomers, such as TOTO, YOYO, TO-PRO, Cy3, Cy5, Cy5.5, Cy7 etc., or dyes such as LCRed 705 may be used as the fluorescent dye.

In particular embodiments detection means are capable of detecting an acoustical signal (quartz crystal microbalance (QCM), surface acoustic waves (SAW) or Bulk Acoustic Wave (BAW) etc.). Such acoustic signals may be generated by liposomes, micelles, bubbles. Such vesicles may be filled with a liquid, a gas, a gaseous precursor, and/or a solid or solute material.

Depending on the nature of the signal to be detected, the detection surface can be an integral part of the detection means (sensor surface) or can allow the detection of the presence of magnetic particles on its surface.

Accordingly another aspect of the present invention provides tools and devices for carrying out the methods of the present invention. The tools and devices comprise a detection surface with immobilised (second) anti-PTH antibodies and means for contacting the detection surface with magnetic particles comprising (first) anti-PTH antibodies. Optionally, the devices and tools comprise means for ensuring magnetic actuation of the magnetic particles. Finally the tools and devices may comprise an integrated detection means.

The methods and tools of the invention can be applied in the diagnosis of parathyroid disease in medical/healthcare applications in the hospital, physician's office and also to enhance the speed of tests done in centralised laboratories. Moreover, the invention can also be used to test for PTH during intra-operative situations for decision making in the OR. PTH is routinely monitored in patients with a kidney disorder called chronic renal failure (CRF). Because PTH is one of the major factors affecting calcium metabolism, the PTH test helps to distinguish nonparathyroid from parathyroid causes of too much calcium in the blood (hypercalcemia).

Accordingly, a further aspect of the present invention relates to methods for detecting parathyroid disease or to evaluate the level of blood calcium by detecting PTH level using the methods and tools of the invention.

Other arrangements of the systems and methods embodying the invention will be obvious for those skilled in the art.

It is to be understood that although preferred embodiments, specific constructions and configurations, as well as materials, have been discussed herein for devices according to the present invention, various changes or modifications in form and detail may be made without departing from the scope and spirit of this invention.

### EXAMPLES

### Example 1. Detection of PTH via GMR with 1-step assay

Affinity purified goat anti-PTH antibodies (binding amino acids 39-84 of human PTH) were immobilised from solution by adsorption on a Au sensor surface. A PTH sample is added to a solution of anti-PTH antibodies (binding AA 1-34 of human PTH) applied as mono layer on 300 nm and 500 nm magnetic particles in 100 mM Sodium borate pH 9.4, 150 mM NaCl, 0.5% BSA, 0.1% goat IgG, 1% Tween 60, at a concentration of 0.25 mg particles/ml. The sample with magnetic particles is exposed to the sensor and an actuation sequence towards the surface of 16 cycles actuation on and then off is applied (total time of approximately 10 minutes). During each on pulse, a frequency of 1 Hz with a duty cycle of 10% with a field of approximately 6x 10⁴ A/m is employed. Thereafter a wash field away from the surface of a similar strength is applied for 2 minutes and the signal change is calculated. Typical settings which may be applied are a frequency between 0.5 and 10Hz, a duty cycle between 5 and 25%, a field of between 1x 10³ A/m to 1x 10⁵ A/m, and 1 to 40 cycles per minute assay time.

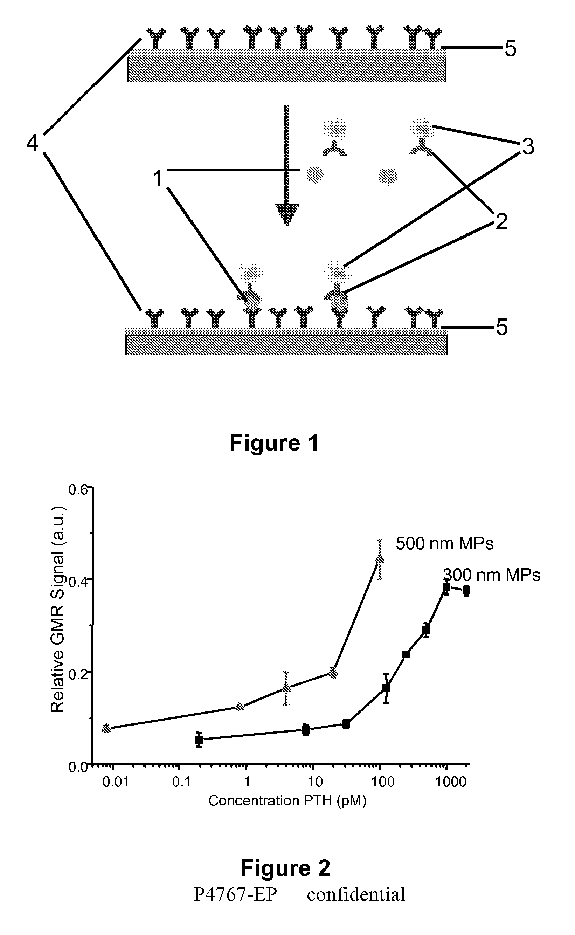

A dose response curve for the detection of the magnetic particles bound to the sensor surface is shown in Figure 2.

### Example 2. Adsorption of antibodies on gold

After the cleaning of an Au surface via oxidative techniques (O₂ plasma, UV/Ozone ...) a solution of anti-PTH in PBS was brought into contact with the surface for more than 1 hour and thereafter washed with PBS or a PBS Tween solution. It was found that high concentrations of antibodies in the adsorption solution was favourable (>0.05 mg/ml).

Using a mixed layer of alkanethiols containing functional COOH groups and EDC/NHS chemistry it is also possible to obtain functional antibodies on the Au surface. It has been observed that an alkanethiol solution containing less than 10% mol of the carboxylated thiol is optimal. It is also desirable for this procedure that the pH of the antibody solution be approximately 5 (e.g. MES) or 9 (e.g. borate) and that the adsorption solution contain a high concentration of antibodies (>0.20 mg/ml).

## Claims

1. A method of detecting parathyroid hormone (PTH) in a sample comprising the steps of:
a) contacting the sample with a soluble, first anti-PTH antibody coupled to a magnetic particle with a diameter between 100 and 1000 nm,
b) manipulating the magnetic particles with a magnetic field so as to bring said particles into contact with a detection surface comprising a second anti-PTH antibody immobilised on said detection surface,
c) detecting the binding of said magnetic particles on the detection surface wherein, steps a) and b) are performed in an alkaline buffer between pH 8.2 and 10.6.

2. The method according to claim 1 wherein the manipulation comprises the repeated application of a magnetic field manipulating magnetic particles towards the sensor surface.

3. The method according to claim 1 or 2, wherein said manipulation comprises the application of an additional magnetic field generating a translational and/or rotational movement of magnetic particles over the sensor surface.

4. The method according to any one of claims 1 to 3, wherein the buffer is a borate buffer.

5. The method according to any one of claims 1 to 4, wherein the buffer further comprises BSA or IgG.

6. The method according to any one of claims 1 to 5 wherein the buffer further comprises a detergent.

7. The method according to claim 6 wherein said detergent is Tween 60.

8. The method according to any one of claims 1 to 7 wherein said detection of said magnetic particles is performed based on the magnetic properties of said particles.

9. The method according to any one of claims 1 to 7, wherein said detection of said magnetic particles is performed based on the optical properties of said particles.

10. The method according to any one of claims 1 to 7, wherein said magnetic particles further comprise an optical label and said detection is performed based on the detection of said optical label.

11. Use of a buffer with a pH of between 8.2 and 10.6 in a sandwich ELISA for PTH involving magnetic particles.

12. Use according to claim 11 wherein said buffer is a borate buffer and further comprises BSA and/or IgG.

13. Use according to claim 11 or 12 wherein said buffer further comprises a detergent.

14. A composition comprising antibodies against PTH, said antibodies labelled with magnetic particles, wherein the magnetic particles have a diameter between 200 and 500 nm.

15. The composition according to claim 14, further comprising PEG or biotin.

16. The composition according to claim 14 or 15, further comprising a buffer with a pH of between 8.2 and 10.6.
